# EUROPÄISCHE PATENTSCHRIFT

(11) **EP 2 311 574 B1**
(45) Veröffentlichungstag und Bekanntmachung des Hinweises auf die Patenterteilung: **09.04.2014**
(21) Anmeldenummer: 10009800.3
(22) Anmeldetag: 17.09.2010
(51) Int. Cl.: B05B 11/00, A61M 15/00, B65D 83/14

(54) **Austragvorrichtung**
Discharge device
Dispositif de sortie

(30) Priorität: 13.10.2009 DE 102009049902
(43) Veröffentlichungstag der Anmeldung: 20.04.2011
(73) Patentinhaber: Aptar Radolfzell GmbH, 78315 Radolfzell (DE)
(72) Erfinder: Thomas, Eberhard, 78315 Radolfzell (DE)
(74) Vertreter: Patentanwälte Ruff, Wilhelm, Beier, Dauster & Partner

(56) Entgegenhaltungen:
- EP-A1- 0 509 863
- WO-A1-03/074189

## Beschreibung

### Anwendungsgebiet und Stand der Technik

Die Erfindung betrifft eine Austragvorrichtung für eine Flüssigkeit, insbesondere eine pharmazeutische Flüssigkeit, zur Aufnahme und Betätigung eines Pumpspenders.

Eine gattungsgemäße Austragvorrichtung weist einen Pumpspender auf, der seinerseits einen Flüssigkeitsspeicher, eine Pumpeinrichtung und ein Auslassstutzen mit Auslasskanal aufweist und dessen Pumpeinrichtung durch ein Niederdrücken des Auslassstutzens in Richtung des Flüssigkeitsspeichers betätigbar ist. Weiterhin weist eine gattungsgemäße Austragvorrichtung einen Aufnahmebereich zur zumindest teilweisen Aufnahme des Pumpspenders auf mit einem Auslassstutzenhalter mit einem Flüssigkeitskanal, wobei der Auslassstutzenhalter und der Auslassstutzen in einer Endrelativlage zur flüssigkeitsführenden und nach außen flüssigkeitsdichten Verbindung des Flüssigkeitskanals mit dem Auslasskanal ausgebildet sind, und mit einer Kraftbeaufschlagungsfläche, die derart angeordnet ist, dass durch eine Verlagerung der Kraftbeaufschlagungsfläche in Richtung des Auslassstutzenhalters auch der Flüssigkeitsspeicher des Pumpspenders in Richtung des Auslassstutzenhalters verlagert werden kann. Des weiteren verfügt eine gattungsgemäße Austragvorrichtung über zwei gegeneinander durch eine Führungseinrichtung geführt verlagerbare Gehäuseabschnitte, wobei der Auslassstutzen an einem dieser Gehäuseabschnitte ortsfest vorgesehen ist und wobei die Kraftbeaufschlagungsfläche an dem anderen der Gehäuseabschnitte ortsfest vorgesehen ist.

Weiterhin ist bei einer gattungsgemäßen Austragvorrichtung vorgesehen, dass in einem Lieferzustand der Austragvorrichtung die beiden Gehäuseabschnitte und damit der Auslassstutzenhalter und die Kraftbeaufschlagungsfläche derart voneinander beabstandet sind, dass der Auslassstutzen in einer von der Endrelativlage beabstandeten Relativlage zum Auslassstutzenhalter angeordnet ist und dass die Gehäuseabschnitte derart gegeneinander verlagerbar sind, dass der Auslassstutzen durch eine Verlagerung der Gehäuseabschnitte in seine Endrelativlage zum Auslassstutzenhalter gebracht werden kann.

Eine gattungsgemäße Austragvorrichtung ist dafür ausgebildet, einen allgemein üblichen Pumpspender aufzunehmen, der seinerseits bereits die wesentlichen Komponenten zur Speicherung und Förderung einer pharmazeutischen Flüssigkeit aufweist. Die den Pumpspender umgebenden Teile der Austragvorrichtung, so wie die beiden Gehäuseabschnitte, dienen der bequemen Handhabung dieses Pumpspenders, der Weiterleitung der durch den Pumpspender geförderten Flüssigkeit zu einer Austragöffnung, der Verwirbelung der Flüssigkeit im Bereich dieser Austragöffnung sowie gegebenenfalls der Bereitstellung einer ergänzenden Funktionalität wie beispielsweise einer durch ein elektronisches Zählmodul bereitgestellten Zählung der bereits getätigten Austragvorgänge.

Zur Betätigung des Pumpspenders sind die beiden Gehäuseabschnitte vorgesehen, wobei bei gattungsgemäßen Austragvorrichtungen üblicherweise ein Gehäuseabschnitt einen Hauptabschnitt bildet, gegenüber dem der andere Gehäuseabschnitt als Betätigungsabschnitt durch die Führungseinrichtung geführt verlagerbar ist. Bestimmungsgemäß wird durch eine Verlagerung der Gehäuseabschnitte eine Verkleinerung des Aufnahmebereichs bewirkt, d.h. die Kraftbeaufschlagungsfläche und der Auslassstutzenhalter, die an gegenüberliegenden Seiten des Aufnahmebereichs angeordnet sind, werden einander angenähert, um die Pumpeinrichtung des Pumpspenders zu betätigen. An jenem Gehäuseabschnitt, der den Auslassstutzenhalter aufweist, ist vorzugsweise auch die bereits genannte Austragöffnung zum Austrag der Flüssigkeit vorgesehen.

Bei bekannten Austragvorrichtungen ist üblicherweise vorgesehen, dass der Auslassstutzen des Pumpspenders und der Auslassstutzenhalter bereits im Lieferzustand ineinandergeschoben und dadurch kraftschlüssig miteinander gekoppelt sind. Die Verbindung des Auslassstutzenhalters und des Auslassstutzens erfolgt demnach bereits zuvor während der Montage durch Eindrücken des Auslassstutzens in den Auslassstutzenhalter. Wenn die Kraftbeaufschlagung hierfür am Flüssigkeitsspeicher des Pumpspenders erfolgt, geht dies jedoch aufgrund der bewirkten Verlagerung des Auslassstutzens gegenüber dem Flüssigkeitsspeicher des Pumpspenders mit einer Erstbetätigung des Pumpspenders einher. Dies wird als nachteilig angesehen, da hierdurch bereits während der Montage der Austragvorrichtung zum Zwecke des Volumenausgleichs Luft in Pumpspender eingesogen wird und somit zu einer Kontaminationswirkung oder Oxidationswirkung führen kann. Eine gegebenenfalls im Pumpspender während dessen Herstellung eingebrachte Schutzgasatmosphäre kann durch diese erste Betätigung während der Montage geschwächt oder zerstört werden.

Eine gattungsgemäße Austragvorrichtung ist aus der EP 0509863 A1 bekannt. Die darin beschriebene Austragvorrichtung weist eine Kappe auf, die nach Abnahme vom Spender auf der gegenüberliegenden Seite des Spenders aufgesetzt werden kann, um dort als Betätigungshandhabe zu agieren. Aus der genannten Schrift ist es auch eine Anordnung bekannt, bei der vor der ersten Betätigung der Auslassstutzen noch nicht in den Auslassstutzenhalter eingefügt ist.

### Aufgabe und Lösung

Aufgabe der Erfindung ist es, eine gattungsgemäße Austragvorrichtung dahingehend weiterzubilden, dass diese in gegenüber dem Stand der Technik vorteilhafter Weise geeignet ist, bis zu Erstbetätigung durch den Patienten unbetätigt zu bleiben, so dass insbesondere eine Schutzgasatmosphäre nicht bereits bei der Montage wieder geschwächt wird. Aufgabe der Erfindung ist es weiterhin, ein korrespondierendes Verfahren zur Montage und Inbetriebnahme einer Austragvorrichtung zur Verfügung zu stellen.

Erfindungsgemäß wird die Aufgabe dadurch gelöst, dass ein erster Gehäuseabschnitt der beiden Gehäuseabschnitte als zweiteiliger Gehäuseabschnitt ausgebildet ist, wobei ein erster Gehäuseteilabschnitt des ersten Gehäuseabschnitts die Kraftbeaufschlagungsfläche bzw. den Auslassstutzenhalter aufweist und ein zweiter Gehäuseteilabschnitt des ersten Gehäuseabschnitts gemeinsam mit dem zweiten Gehäuseabschnitt die Führungseinrichtung zum Zweck der geführten Beweglichkeit der Gehäuseteile gegeneinander aufweist. Dabei sind die beiden Gehäuseteilabschnitte gegeneinander verlagerbar aneinander angebracht.

Bei einem erfindungsgemäßen Spender ist vorgesehen, dass der Aufnahmebereich, also der Bereich zwischen dem Auslassstutzenhalter und der Kraftbeaufschlagungsfläche, im Lieferzustand eine Größe aufweist, die es gestattet, dass der Pumpspender im unbetätigten Zustand darin Platz findet, ohne dass der Auslassstutzen hierfür bereits in seiner Endrelativlage relativ zum Auslassstutzenhalter angeordnet sein muss. Der Pumpspender kann vollständig oder teilweise im Aufnahmebereich angeordnet sein. Erheblich ist allerdings, dass in diesem Lieferzustand die Kraftbeaufschlagungsfläche relativ zu einem kraftbeaufschlagbaren Teil des Flüssigkeitsspeichers bereits derart angeordnet ist, dass eine Verlagerung der Kraftbeaufschlagungsfläche in Richtung des Auslassstutzenhalters zu einem Berührkontakt der Kraftbeaufschlagungsfläche mit diesem Teil des Flüssigkeitsspeichers führt, so dass die Verlagerung der Kraftbeaufschlagungsfläche es gestattet, den Auslassstutzen des Pumpspenders in den Auslassstutzenhalter hineinzudrücken. Der Lieferzustand ist dabei jener Zustand, in dem die Austragvorrichtung an den Endkunden, also an den Patienten, ausgeliefert wird, üblicherweise mit zusammen mit einer Umverpackung wie einer Faltschachtel und mit einem Beipackzettel mit Arzneimittelhinweisen.

Die erforderliche Kraft zur Verbringung des Auslassstutzens in diese Endrelativlage wird nicht während der Montage der Austragvorrichtung aufgebracht, sondern stattdessen erst durch den Patienten, vorzugsweise im Zuge der Erstbetätigung der Austragvorrichtung, mittels einer Verlagerung der Kraftbeaufschlagungsfläche gegenüber dem Auslassstutzenhalter. Wenn dies durch die Verlagerung der Gehäuseabschnitte gegeneinander erfolgt, die bestimmungsgemäß im Betrieb zum Hervorrufen eines Austragvorgangs gegeneinander verlagert werden, ist der Vorgang der Inbetriebnahme durch den Patienten gegenüber herkömmlichen Austragvorrichtungen nahezu unverändert. Die gleiche Art der Betätigung, die bereits bei gattungsgemäßen Austragvorrichtungen zum Austrag der Flüssigkeit dient, dient bei einer erfindungsgemäßen Austragvorrichtung im Zuge der Erstbestätigung sowohl der Verbringung des Auslassstutzens in seine Endrelativlage zum Auslassstutzenhalter als auch der Bewirkung der Erstbetätigung des Pumpspenders.

Die Gehäuseabschnitte sind gegeneinander durch eine Führungseinrichtung geführt. Vorzugsweise ist diese Führungseinrichtung zur translativen Führung entlang einer Haupterstreckungsrichtung der Austragvorrichtung ausgebildet, welche mit einer Betätigungsrichtung des Pumpspenders übereinstimmt.

Durch die erfindungsgemäße Gestaltung ist es möglich, die Führungseinrichtung zwischen den Gehäuseabschnitten frei von besonderen Vorkehrungen zur Limitierung der Bewegungsfreiheit nach der Inbetriebnahme freizuhalten. Die Führungseinrichtung kann demnach unveränderlich dafür ausgelegt sein, lediglich eine solche Relativverlagerung der Gehäuseabschnitte zuzulassen, die in etwa dem Pumphub der Pumpeinrichtung des Pumpspenders entspricht. Die zusätzliche Verlagerungsstrecke, die erforderlich ist, damit im Lieferzustand der Auslassstutzen von der Endrelativlage relativ zum Auslassstutzenhalter beabstandet sein kann, wird durch die Zweiteilung des ersten Gehäuseabschnitts erzielt. Im Lieferzustand befinden sich die beiden Gehäuseteilabschnitte des ersten Gehäuseabschnitts demnach in einer ersten Relativlage, die im Zuge der Inbetriebnahme in eine zweite Relativlage überführt wird. Die Relativverlagerung erfolgt vorzugsweise in gleicher Richtung, in der die Führungseinrichtung die beiden Gehäuseabschnitte gegeneinander führt.

Zur erleichterten Verlagerung der Gehäuseabschnitte gegeneinander weisen diese vorzugsweise jeweils Fingerauflageflächen auf, wobei insbesondere vorzugsweise der Gehäuseabschnitt mit der Austragöffnung zwei beidseitig der Austragöffnung vorgesehene Fingerauflagen für Zeigefinger und Mittelfinger aufweist und der Gehäuseabschnitt mit der Kraftbeaufschlagungsfläche eine Fingerauflage für den Daumen aufweist. Es ist jedoch nicht zwingend erforderlich, dass beide Gehäuseabschnitte unmittelbar für eine Kraftbeaufschlagung durch den Patienten zugänglich sind. Es sind auch Ausführungsformen von der Erfindung umfasst, bei denen zumindest einer der Gehäuseabschnitte nur mittelbar verlagert werden kann, beispielsweise durch eine am Hauptgehäuseabschnitt angebrachte Handhabe, die bei Betätigung die Verlagerung des in diesem Fall als Innengehäuseabschnitts innerhalb der Hauptgehäuseabschnitts ausgebildeten Gehäuseabschnitts mittelbar bewirkt. So kann eine Betätigungsrichtung einer solchen separaten Handhabe von einer Bewegungsrichtung des Gehäuseabschnitts abweichen.

Zur sicheren Erzielung und Aufrechterhaltung der flüssigkeitsführenden und nach außen flüssigkeitsdichten Verbindung zwischen dem Auslassstutzenhalter und dem Auslassstutzen in deren Endrelativlage ist es vorzugsweise vorgesehen, dass der Auslassstutzen und der Auslassstutzenhalter in der Endrelativlage kraftschlüssig aneinander gehalten werden, wobei eine erforderliche Trennkraft größer ist als die Gewichtskraft des Pumpspenders. Somit bleibt die im Zuge der Inbetriebnahme erzielte Kopplung des Auslassstutzens an den Auslassstutzenhalter während der gesamten auf die Inbetriebnahme folgenden Lebensdauer der Austragvorrichtung oder zumindest des Pumpspenders erhalten. Als besonders vorteilhaft wird es angesehen, wenn der Auslassstutzen und der Auslassstutzenhalter korrespondierend zueinander Zylinderabschnitte aufweisen, wobei der Außendurchmesser des Zylinderabschnitts des Auslassstutzens und der Innendurchmesser des Zylinderabschnitts des Auslassstutzenhalters für eine kraftschlüssige Pressverbindung in der Endrelativlage ausgebildet sind und wobei die Zylinderabschnitte im Lieferzustand vorzugsweise nicht im Eingriff miteinander sind. Allerdings sind auch Gestaltungen denkbar, bei denen der Innendurchmesser des Auslassstutzens und der Außendurchmesser des Auslassstutzenhalters zur Erzielung einer solchen Pressverbindung ausgebildet sind. Die Kraft zur Erzielung der Pressverbindung ist durch den Patienten aufzubringen und liegt daher vorzugsweise unterhalb von 50 Newton.

Um einen sicheren Eingriff der Zylinderabschnitte ineinander zu erzielen, kann eine Einführschräge an einem der Zylinderabschnitte oder an beiden Zylinderabschnitten vorgesehen sein.

Die Endrelativlage ist jene Relativlage des Auslassstutzens zum Auslassstutzenhalter, die im Normalbetrieb vorgesehen ist. Zur Definition der Endrelativlage ist vorzugsweise am Auslassstutzenhalter ein Anschlag vorgesehen, an dem der Auslassstutzen im Zuge der Kopplung zum Anliegen kommt. Dieser Anschlag gewährleistet, dass eine Verlagerung der Kraftbeaufschlagungsfläche gegenüber dem Auslassstutzenhalter einen definierten Pumpenhub bewirkt, da die Endrelativlage des Auslassstutzens und des Auslassstutzenhalters besonders klar definiert ist.

Besonders zweckmäßig ist die Verwendung einer erfindungsgemäßen Austragvorrichtung im Zusammenhang mit einer im Pumpspender im Lieferzustand bestehenden Schutzgasatmosphäre, insbesondere einer Stickstoffschutzgasatmosphäre oder eine Kohlendioxidschutzgasatmosphäre. Diese Schutzgasatmosphäre, die insbesondere oxidationshemmend oder dekontaminierend wirken kann, wird während der Abfüllung des Pumpspenders in diesen eingebracht. Durch die erfindungsgemäße Gestaltung der Austragvorrichtung wird die Schutzgasatmosphäre erst dann geschwächt, wenn der Patient die Inbetriebnahme durch die Erstbetätigung vornimmt. Ebenfalls zweckmäßig ist die Verwendung der erfindungsgemäßen Austragvorrichtung mit einem Pumpspender, bei dem die Pumpeinrichtung über einen Verbindungskanal mit dem Flüssigkeitsspeicher verbunden ist und wobei die Flüssigkeit im Lieferzustand lediglich im Flüssigkeitsspeicher selbst und im Verbindungskanal angeordnet ist. Bei einer solchen Gestaltung, bei der die Flüssigkeit im Lieferzustand primär im Flüssigkeitsspeicher vorgesehen ist und insbesondere noch nicht bis zum Pumpeinrichtung gelangt ist, ist nicht zu befürchten, dass das Zusammenwirken der Flüssigkeit mit Teilen der Pumpeinrichtung, beispielsweise metallischen Teilen, zu einer nachteiligen Wirkung führt. Die Pumpeinrichtung kommt erst in Kontakt mit der Flüssigkeit, wenn die Inbetriebnahme der Austragvorrichtung im Zuge der Erstbetätigung durch den Patienten stattfindet.

Die Gehäuseabschnitte sind vorzugsweise derart ausgebildet, dass die Maximalbeabstandung zwischen der Kraftbeaufschlagungsfläche und dem Auslassstutzenhalter nach einer Erstbetätigung geringer ist als die Beabstandung zwischen der Kraftbeaufschlagungsfläche und dem Auslassstutzenhalter im Lieferzustand. Der Aufnahmebereich für den Pumpspender nimmt demnach nach der Inbetriebnahme durch Erstbetätigung der Austragvorrichtung nicht wieder die gleiche Größe ein, die er zuvor im Lieferzustand gehabt hat. Hierdurch ist gewährleistet, dass der Auslassstutzen des Pumpspenders sich nach der Inbetriebnahme nicht wieder vom Auslassstutzenhalter lösen kann. Vorzugsweise wird dieses Verhalten durch Rastmittel bewirkt, die eine Rückkehr der Kraftbeaufschlagungsfläche in ihre Relativstellung zum Auslassstutzenhalter des Lieferzustandes nach Erstinbetriebnahme verhindern.

Vorzugsweise sind korrespondierend zueinander Anschlagseinrichtungen am zweiten Gehäuseteilabschnitt des ersten Gehäuseabschnitts und am zweiten Gehäuseabschnitt vorgesehen, die formschlüssig eine Trennung des zweiten Formteilabschnitts vom zweiten Gehäuseabschnitt verhindern. Diese Anschlagseinrichtungen sind derart ausgebildet und/oder angeordnet, dass sie eine Relativverlagerung des zweiten Gehäuseteilabschnitts gegenüber dem zweiten Gehäuseabschnitt gestatten, die in etwa dem Pumpenhub der Pumpeinrichtung des Pumpspenders entspricht.

Besonders bevorzugt ist eine Gestaltung, bei der am zweiten Gehäuseteilabschnitt mindestens ein Sperrabschnitt vorgesehen ist, der in eine Ausnehmung einer Wandung des zweiten Gehäuseabschnitts hineinragt oder durch diese Ausnehmung hindurchragt und der eine Verlagerung des zweiten Gehäuseteilabschnitts verhindert, solange eine Kappe auf dem zweiten Gehäuseteilabschnitt aufgesetzt ist. Gerade im Hinblick auf das Vorhandensein eines solchen Sperrabschnitts ist es von Vorteil, wenn der erste Gehäuseabschnitt zweiteilig ausgebildet ist, da in einem solchen Fall der zweite Gehäuseteilabschnitt bereits im Lieferzustand so angeordnet sein kann, dass der Sperrabschnitt bis in die Ausnehmung in der Wandung des zweiten Gehäuseabschnitts hineinragt oder unmittelbar vor dieser positioniert ist. Zweck des Sperrabschnitts ist es, eine ungewollte Betätigung der Austragvorrichtung dadurch zu verhindern, dass eine Verlagerung des zweiten Gehäuseabschnitts gegenüber dem ersten Gehäuseabschnitt, die einen Austragvorgang bewirken würde, erst bei abgenommener Kappe möglich ist.

Bei der Gestaltung mit zwei Gehäuseteilabschnitten wird es als besonders bevorzugt angesehen, wenn die Gehäuseteilabschnitte zueinander korrespondierende Rastmittel aufweisen, durch die sie in zwei unterschiedliche Relativlagen miteinander verrastbar sind. Dabei entspricht eine erste Relativlage der Gehäuseteilabschnitte dem Lieferzustand und eine zweite Relativlage dem nach der Inbetriebnahme durch die Erstbetätigung erzielten und ab diesem Zeitpunkt aufrechterhaltenen Betriebszustand. Die Relativstellung des ersten Gehäuseteilabschnitts zum zweiten Gehäuseteilabschnitt unterscheidet sich zwischen den beiden Relativlagen vorzugsweise in etwa um jene Distanz, um die der Auslassstutzen im Lieferzustand von seiner Endrelativlage gegenüber dem Auslassstutzenhalter beabstandet ist.

Die Erfindung betrifft weiterhin ein Verfahren zur Montage und Inbetriebnahme einer Austragvorrichtung des oben beschriebenen Typs, wobei in einem Montageschritt die Gehäuseabschnitte soweit ineinander eingeschoben werden, dass die Kraftbeaufschlagungsfläche ausreichend weit vom Auslassstutzenhalter beabstandet ist, dass der Auslassstutzen bei Anliegen des Pumpspenders an der Kraftbeaufschlagungsfläche noch nicht in einer Endrelativlage relativ zum Auslassstutzenhalter angeordnet ist, und wobei in einem dem Montageschritt nachgeschalteten Inbetriebnahmeschritt nach der Auslieferung an den Patienten durch diesen durch fortgesetztes Einschieben der Gehäuseabschnitte ineinander der Auslassstutzen bis in seine Endrelativlage relativ zum Auslassstutzen gebracht wird.

### Kurzbeschreibung der Zeichnungen

Weitere Aspekte und Vorteile der Erfindung ergeben sich außer aus den Ansprüchen auch aus der nachfolgenden Beschreibung eines bevorzugten Ausführungsbeispiels der Erfindung, welches nachfolgend anhand der Figuren erläutert wird. Dabei zeigen:
- Fig. 1: eine Explosionsdarstellung einer erfindungsgemäßen Austragvorrichtung und
- Fig. 2a bis 2e: Die Austragvorrichtung der Fig. 1 in verschiedenen Stadien vom Lieferzustand über die Inbetriebnahme bis zum gesicherten Zustand.

### Detaillierte Beschreibung des Ausführungsbeispiels

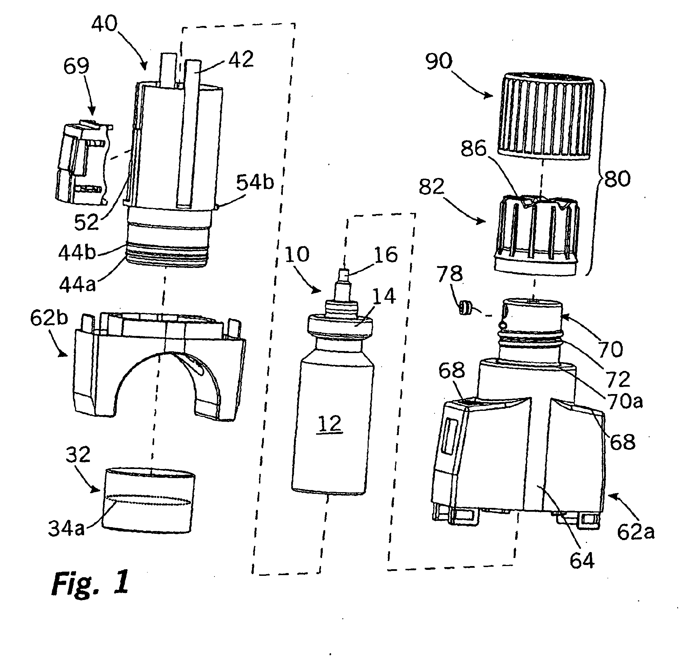

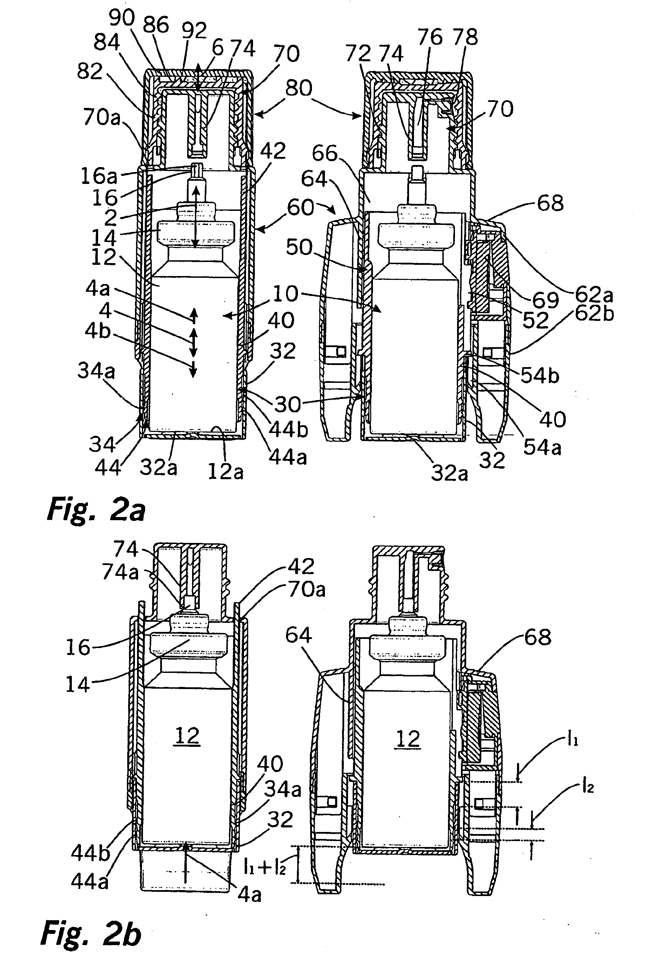

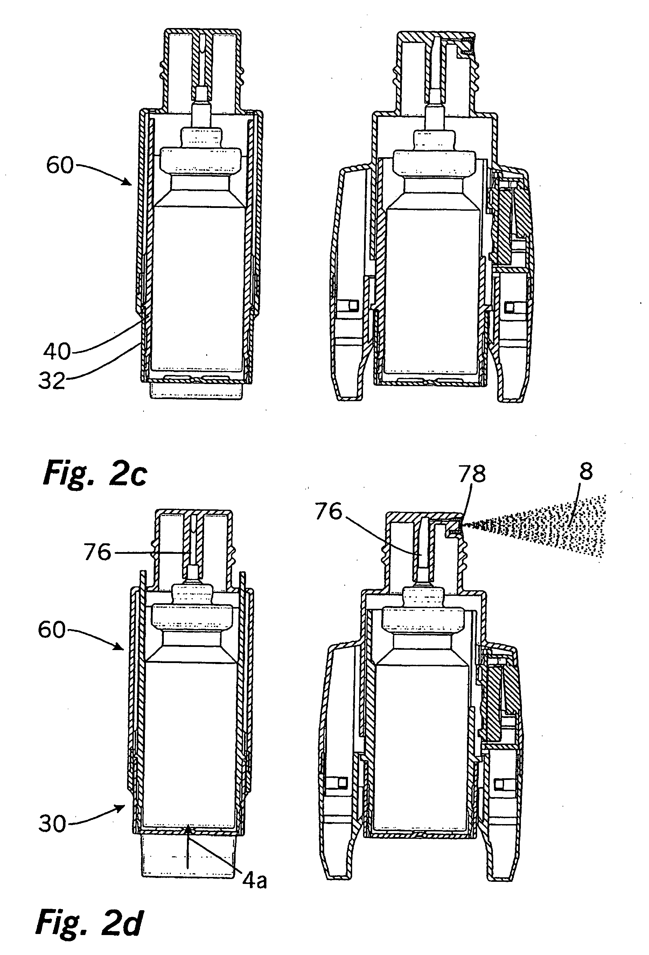

Unter Bezugnahme auf die Fig. 1 und 2a wird nachfolgend zunächst der Aufbau des dargestellten Ausführungsbeispiels erläutert.

Die erfindungsgemäße Austragvorrichtung umfasst einen Pumpspender 10 sowie zwei Gehäuseabschnitte 30, 60 und eine Kappe 80.

Der Pumpspender 10 stellt einen handelsüblichen Pumpspender mit einem Flüssigkeitsspeicher 12, einer nicht näher dargestellten Pumpeinrichtung 14 sowie einem Auslassstutzen 16 dar. Der Auslassstutzen 16 ist in Pumprichtung 2 gegenüber dem Flüssigkeitsspeicher 12 verlagerbar, um dadurch eine Betätigung der Pumpeinrichtung 14 und einen Austrag von Flüssigkeit aus dem Flüssigkeitsspeicher 12 durch einen Auslasskanal 16a des Auslassstutzens 16 zu bewirken.

Die beiden Gehäuseabschnitte 30, 60 sind dafür ausgebildet, im Betrieb durch eine Führung 50, die im Folgenden noch erläutert wird, in Richtung des Pfeils 4 gegeneinander beweglich zu sein.

Der Gehäuseabschnitt 60 bildet einen Hauptabschnitt 60 der dargestellten Austragvorrichtung. Er besteht aus zwei Teilen 62a, 62b, die bereits während der Montage aneinander angebracht werden und in einer festen Relativlage zueinander verbleiben, so dass sie im Weiteren gemeinsam als Hauptabschnitt 60 auf sie Bezug genommen wird. Dieser Hauptabschnitt 60 weist einen in etwa zylindrischen Innenabschnitt 64 auf, der einen Aufnahmeraum 66 umgibt. Seitlich ragen von diesem Innenabschnitt 64 zwei Fingerauflagen 68 weg, wobei unterhalb der einen der beiden Fingerauflagen ein Aufnahmebereich für ein elektronisches Zählermodul 69 vorgesehen ist. Nach unten hin ist der Hauptabschnitt 60 zum Zwecke der Handhabbarkeit des aufgenommenen Pumpspenders 10 und des ersten Gehäuseabschnitts 30 offen ausgebildet. Auf der gegenüberliegenden oberen Seite weist der Hauptkörper 60 einen Sprühkopf 70 auf, an dessen Außenseite ein Außengewinde 72 angeformt ist. Innerhalb des Sprühkopfs 70 ist ein Auslassstutzenhalter 74 zur Aufnahme des Auslassstutzens 16 des Pumpspenders 10 vorgesehen. Im Auslassstutzenhalter 74 ist ein Flüssigkeitskanal 76 vorgesehen, der den Auslassstutzenhalter 74 mit einer Austragöffnung 78 verbindet.

Der andere Gehäuseabschnitt 30 bildet einen Betätigungsabschnitt 30, der seinerseits einen ersten Gehäuseteilabschnitt 32 und einen zweiten Gehäuseteilabschnitt 40 aufweist. Der zweite Gehäuseteilabschnitt 40 ist etwa rohrförmig ausgebildet und hinsichtlich seines Innendurchmessers zur Aufnahme des Pumpspenders 10 ausgebildet. An diesem Rohrabschnitt 40 sind Fortsätze 42 angeformt, deren Ende sich im Lieferzustand unmittelbar vor korrespondierenden Durchbrechungen 70a des Hauptabschnitts 60 befinden. Auf seiner dem Auslassstutzenhalter 74 abgewandten Seite ist der erste Gehäuseteilabschnitt 32 des Betätigungsabschnitts 30 vorgesehen, der als becherförmiger Bodenabschnitt 32 ausgebildet ist. Dieser Bodenabschnitt 32 ist auf das Ende des Rohrabschnitts 40 aufgeschoben.

Die Kappe 80 ist zweiteilig ausgebildet und weist einen Innenabschnitt 82 mit einem Innengewinde 84 auf. Auf diesen im aufgesetzten Zustand für den Patienten nicht zugänglichen Innenabschnitt 82 ist ein Außenabschnitt 90 aufgesetzt, der gegenüber dem Innenabschnitt 82 in Richtung des Pfeils 6 verlagerbar ist, wobei eine Stirnseite 86 des Innenabschnitts 82 und die Innenseite 92 des Außenabschnitts 90 zum formschlüssigen und drehfesten Koppeln ausgebildet sind, wenn der Außenabschnitt 90 an den Innenabschnitt 82 angepresst wird.

Wie bereits erwähnt wurde, ist eine Führungseinrichtung 50 vorgesehen, die eine Relativbewegung des Rohrabschnitts 40 gegenüber dem Innenabschnitt 64 des Hauptabschnitts 60 gestattet und begrenzt. Diese Führungseinrichtung 50 wird primär durch die Anpassung des Außendurchmessers des Rohrabschnitts 40 an den Innendurchmesser des Innenabschnitts 64 gebildet, durch die die Relativbeweglichkeit in Richtung des Pfeils 4 gegeben ist. Weiterhin wird durch einen radial nach außen tretenden Fortsatz 52 am Rohrabschnitt 40 gewährleistet, dass eine Drehbeweglichkeit um eine durch den Pfeil 4 definierte Achse des Rohrabschnitts 40 gegenüber dem Innenabschnitt 64 nicht möglich ist. Den Führungsmitteln sind weiterhin Anschläge 54a, 54b zugeordnet. Der Anschlag 54b ist als radial nach außen ragender Fortsatz am Rohrabschnitt 40 vorgesehen. Gemeinsam mit dem am Teil 62a vorgesehenen Anschlag 54a begrenzt der Anschlag 54b die Beweglichkeit des Rohrabschnitts 40 in Richtung des Pfeils

Wie oben bereits erläutert, ist auch der Bodenabschnitt 32 gegenüber dem Rohrabschnitt 40 beweglich. Dabei sind sowohl am Rohrabschnitt 40 als auch am Bodenabschnitt 32 miteinander zusammenwirkende Rastmittel 34, 44 vorgesehen. Die Rastmittel 34, 44 umfassen einen nach innen gewandten Rastring 34a am Bodenabschnitt 32 und zwei nach außen offene Nuten 44a, 44b am Rohrabschnitt 40. Der Bodenabschnitt 32 kann somit in Abhängigkeit von der Nut 44a, 44b, in der Rastring 34a angeordnet ist, in zwei unterschiedlichen Relativlagen zum Rohrabschnitt 40 an diesem lagegesichert werden.

Bezugnehmend auf die Fig. 2a bis 2e wird nachfolgend die Inbetriebnahme durch den Patienten erläutert

Fig. 2a zeigt den Lieferzustand der Austragvorrichtung. In diesem Lieferzustand ist die Kappe 80 vollständig auf den Hauptabschnitt 60 der Austragvorrichtung aufgeschraubt. Der Rohrabschnitt 40 ist in den Innenabschnitt 64 des Hauptabschnitts 60 eingeschoben, befindet sich in seiner unteren Endlage, so dass der Anschlag 54b am Anschlag 54a anliegt. Der Bodenabschnitt 32 des Betätigungsabschnitts 30 befindet sich in seiner unteren ersten Relativlage zum Rohrabschnitt 40, die durch den Eingriff des Rastrings 34a in der unteren Rastnut 44a gekennzeichnet ist. Der Pumpspender 10 befindet sich im unbetätigten Zustand. Eine untere Abschlussfläche 12a des Flüssigkeitsspeichers 12 liegt auf einer Kraftbeaufschlagungsfläche 32a des Becherabschnitts 32 auf. Diese Kraftbeaufschlagungsfläche 32a ist aufgrund der Relativanordnung des Bodenabschnitts 32 zum Rohrabschnitt 40 und aufgrund der Relativanordnung des Rohrabschnitts 40 zum Innenabschnitt 64 des Hauptabschnitts 60 so weit vom Auslassstutzenhalter 74 beabstandet, dass der Auslassstutzen 16 des Pumpspenders 10 sich noch nicht im kraftschlüssigen Eingriff mit dem Auslassstutzenhalter 74 befindet.

In nicht dargestellter Weise ist zu diesem Zeitpunkt im Pumpspender 10 noch eine Schutzgasatmosphäre vorhanden, die während der Herstellung des Pumpspenders 10 eingebracht wurde und die bis zu dem in Fig. 2a verdeutlichten Zeitpunkt nicht beschädigt wurde, da eine erste Verlagerung des Auslassstutzens 16 gegenüber dem Flüssigkeitsspeicher 12 zu diesem Zeitpunkt noch nicht stattgefunden hat.

Zum Zwecke der Inbetriebnahme wird ausgehend vom Zustand der Fig. 2a zunächst die Kappe 80 entfernt. Sobald dies geschehen ist, wird die Erstbetätigung durchgeführt, wobei der Patient hierfür seinen Zeigefinger und Mittelfinger auf die Fingerauflagen 68 auflegt und mittels des Daumens den Bodenabschnitt 32 des Betätigungsabschnitts 30 in Richtung des Pfeils 4a nach oben drückt.

Hierdurch werden zwei verschiedene Relativverlagerungen erzielt.

Zum einen kommt es zu einer Relativverlagerung des Betätigungsabschnitts 30 in seiner Gesamtheit gegenüber dem Innenabschnitt 64 um die Distanz l₁. Diese Relativverlagerung ist deshalb möglich, da aufgrund des Abnehmens der Kappe 80 die Fortsätze 42 am Rohrabschnitt 40 durch die Ausnehmungen 70a in jenen Bereich hindurchgleiten können, in dem zuvor die Kappe 80 angeordnet war. Zum anderen kommt es zu einer Relativverlagerung des Bodenabschnitts 32 gegenüber dem Rohrabschnitt 40 um die Distanz l₂. Dabei gleitet der Rastring 34a unter Einwirkung der Betätigungskraft aus der ersten Rastnut 44a heraus und in die zweite Rastnut 44b des Rohrabschnitts 40 hinein.

Gemeinsam mit dem Bodenabschnitt 32 wird auch der Flüssigkeitsspeicher 12 des Pumpspenders 10 gegenüber dem Rohrabschnitt 40 in Richtung des Pfeils 4a um die Distanz I₂ angehoben. Gleichzeitig wird durch die Verlagerung des Rohrabschnitts 40 gegenüber dem Innenabschnitt 64 der Flüssigkeitsspeicher 12 ebenfalls um die Distanz I₁ gegenüber dem Innenabschnitt 64 verlagert. Relativ zum Innenabschnitt 64 ergibt sich somit bei dieser Erstbetätigung im Zuge der Inbetriebnahme eine Verlagerung des Flüssigkeitsspeichers 12 um die Distanz I₁ + I₂.

Diese Verlagerung des Flüssigkeitsspeichers 12 des Pumpspenders 10 um die Distanz I₁ + I₂ bewirkt folgendes: Zunächst wird der Auslassstutzen 16 bis an die Einführschräge 74a des Auslassstutzenhalters 74 herangeführt, ohne jedoch bereits in diesen einzudringen. Es folgt eine Relativverlagerung des Auslassstutzens 16 gegenüber dem Flüssigkeitsspeicher 12, durch die erstmalig ein Pumpvorgang der Pumpeinrichtung 14 bewirkt wird. Sobald die Pumpeinrichtung ihre betätigte Endlage erreicht hat, kann der Auslassstutzen 16 nicht mehr gegenüber dem Flüssigkeitsspeicher 12 verlagert werden und dringt daher in den Auslassstutzenhalter 74 ein, wobei aufgrund der aneinander angepassten Durchmesser 16b des Auslassstutzens 16 und 74a des Auslassstutzenhalters 74 eine kraftschlüssig haltende Pressverbindung zustande kommt. Zu einem Flüssigkeitsaustrag kommt es im Zuge dieser Erstbetätigung nicht, da zunächst nur die Luft aus der Pumpkammer der Pumpeinrichtung 14 verdrängt wird.

Damit ist der Zustand der gedrückte Fig. 2b erreicht und die Inbetriebnahme der Austragvorrichtung abgeschlossen.

Sobald der Patient nun die Kraftbeaufschlagung des Becherabschnitts 32 in Richtung des Hauptabschnitts 60 beendet, kehrt der Pumpspender 10 in seinen unbetätigten Zustand zurück und verlagert dabei in der in Fig. 2c dargestellten Weise den Rohrabschnitt 40 wieder in dessen Ausgangsstellung der Fig. 2a, also bis zu einer Lage, in der die Anschläge 54a, 54b aneinander anliegen. Eine Rückverlagerung des Bodenabschnitts 32 relativ zum Rohrabschnitt 40 findet jedoch nicht statt, da bereits die Verlagerung des Rohrabschnitts 40 gegenüber dem Hauptabschnitt 60 dem vollständigen Pumphub der Pumpeinrichtung 14 entspricht.

Wenn nun eine erneute Betätigung ausgehend vom Zustand der Fig. 2c durch Kraftbeaufschlagung des nunmehr in sich unbeweglichen Betätigungsabschnitts 30 gegenüber dem Hauptabschnitt 60 folgt, kommt es zu einem Austragvorgang von Flüssigkeit aus dem Flüssigkeitsspeicher 12 durch den Kanal 76 bis zur Austragöffnung 78, von der die Flüssigkeit in Form eines Sprühstrahls 8 abgegeben wird. Sobald nach der Betätigung die Kraftbeaufschlagung des Betätigungsabschnitts 30 in Richtung des Pfeils 4a gegenüber dem Hauptabschnitt 60 entfällt, kehrt die Austragvorrichtung in ihrem Zustand der Fig. 2c zurück.

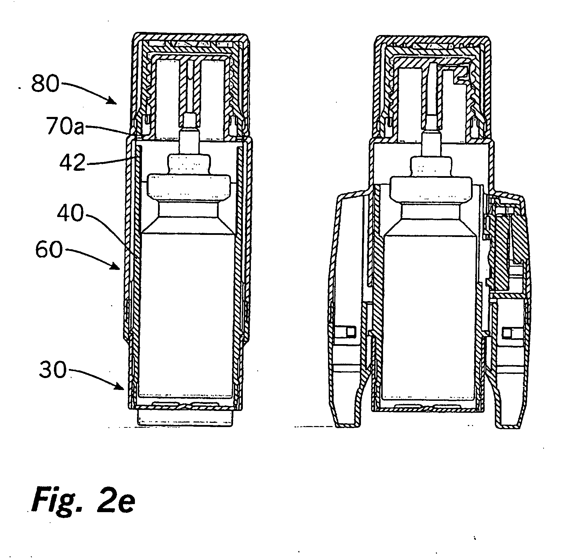

Wie Fig. 2e verdeutlicht, kann die in Betrieb genommene Austragvorrichtung durch Aufsetzen der Kappe 80 vor einer ungewollten Betätigung und einem ungewollten Austragvorgang geschützt werden. Sobald die Kappe 80 aufgesetzt wurde, ist eine Relativverlagerung des Betätigungsabschnitts 30 gegenüber dem Hauptabschnitt 60 nicht mehr möglich, da die am Rohrabschnitt 40 angeformten Sperrfortsätze 42 nicht durch die Ausnehmungen 70a hindurch gleiten können, weil diese von der Kappe 80 blockiert werden. Der Zustand der Fig. 2e verhindert somit beispielsweise einen durch ein Kind bewirkten Austragvorgang, sofern das Kind nicht in der Lage ist, die Sicherheitskappe 80 von der Austragvorrichtung abzunehmen.

## Patentansprüche

1. Austragvorrichtung für eine Flüssigkeit, insbesondere eine pharmazeutische Flüssigkeit, zur Aufnahme und Betätigung eines Pumpspenders (10) mit
- einem Pumpspender (10), der einen Flüssigkeitsspeicher (12), eine Pumpeinrichtung (14) und einen Auslassstutzen (16) mit Auslasskanal (16a) aufweist und dessen Pumpeinrichtung (14) durch ein Niederdrücken des Auslassstutzens (16) in Richtung des Flüssigkeitsspeichers (12) betätigbar ist,
- einem Aufnahmebereich (66) zur zumindest teilweisen Aufnahme des Pumpspenders (10) mit
- einem Auslassstutzenhalter (74) mit einem Flüssigkeitskanal (76), wobei der Auslassstutzenhalter (74) und der Auslassstutzen (16) in einer Endrelativlage zur flüssigkeitsführenden und nach außen flüssigkeitsdichten Verbindung des Flüssigkeitskanals (76) mit dem Auslasskanal (16a) ausgebildet sind, und
- einer Kraftbeaufschlagungsfläche (32a), die derart angeordnet ist, dass durch eine Verlagerung der Kraftbeaufschlagungsfläche (32a) in Richtung (4a) des Auslassstutzenhalters (74) auch der Flüssigkeitsspeicher (12) des Pumpspenders (10) in Richtung des Auslassstutzenhalters (74) verlagert wird, und
- zwei gegeneinander durch eine Führungseinrichtung geführt verlagerbaren Gehäuseabschnitten (30, 60), wobei der Auslassstutzenhalter (74) an einem der Gehäuseabschnitte (60) ortsfest vorgesehen ist und wobei die Kraftbeaufschlagungsfläche (32a) an einem anderen der Gehäuseabschnitte (30) ortsfest vorgesehen ist,
wobei
- in einem Lieferzustand die beiden Gehäuseabschnitte (30, 60) und damit der Auslassstutzenhalter (74) und die Kraftbeaufschlagungsfläche (32a) derart voneinander beabstandet sind, dass der Auslassstutzen (16) in einer von der Endrelativlage beabstandeten Relativlage zum Auslassstutzenhalter (74) angeordnet ist, und
- die Gehäuseabschnitte (30, 60) derart gegeneinander verlagerbar sind, dass der Auslassstutzen (16) durch eine Verlagerung der Gehäuseabschnitte (30, 60) in seine Endrelativlage zum Auslassstutzenhalter (74) gebracht werden kann, **dadurch gekennzeichnet, dass**
- ein erster Gehäuseabschnitt (30) der beiden Gehäuseabschnitte (30, 60) als zweiteiliger Gehäuseabschnitt (30) ausgebildet ist, wobei
- ein erster Gehäuseteilabschnitt (32) des ersten Gehäuseabschnitts (30) die Kraftbeaufschlagungsfläche (32a) bzw. den Auslassstutzenhalter aufweist und ein zweiter Gehäuseteilabschnitt (40) des ersten Gehäuseabschnitts (30) gemeinsam mit dem zweiten Gehäuseabschnitt (60) die Führungseinrichtung (50) zum Zwecke der geführten Beweglichkeit der Gehäuseteile (30, 60) gegeneinander aufweist und
- die beiden Gehäuseteilabschnitte (32, 40) gegeneinander verlagerbar aneinander angebracht sind.

2. Austragvorrichtung nach Anspruch 1,
**dadurch gekennzeichnet, dass**
der Auslassstutzen (16) und der Auslassstutzenhalter (74) korrespondierend zueinander Zylinderabschnitte aufweisen, wobei der Außendurchmesser (16b) des Zylinderabschnitts des Auslassstutzens (16) und der Innendurchmesser (74a) des Zylinderabschnitts des Auslassstutzenhalters (74) für eine kraftschlüssige Pressverbindung in der Endrelativlage ausgebildet sind und wobei die Zylinderabschnitte im Lieferzustand nicht im Eingriff miteinander sind.

3. Austragvorrichtung nach einem der vorstehenden Ansprüche, **dadurch gekennzeichnet, dass**
am Auslassstutzenhalter (74) ein die Endrelativlage definierenden Anschlag vorgesehen ist.

4. Austragvorrichtung nach einem der vorstehenden Ansprüche, **dadurch gekennzeichnet, dass**
- im Lieferzustand im Pumpspender (10) eine Schutzgasatmosphäre besteht, vorzugsweise eine Stickstoff-Schutzgasatmosphäre und/oder
- die Pumpeinrichtung (14) des Pumpspenders (10) über einen Verbindungskanal mit dem Flüssigkeitsspeicher (12) des Pumpspenders (10) verbunden ist, wobei die Flüssigkeit im Lieferzustand lediglich im Flüssigkeitsspeicher (12) selbst und im Verbindungskanal angeordnet ist.

5. Austragvorrichtung nach einem der vorstehenden Ansprüche, **dadurch gekennzeichnet, dass**
die Gehäuseabschnitte (30, 60) derart ausgebildet sind, dass die Maximalbeabstandung zwischen der Kraftbeaufschlagungsfläche (32a) und dem Auslassstutzenhalter (74) nach einer Erstbetätigung geringer ist als die Beabstandung zwischen der Kraftbeaufschlagungsfläche (32a) und Auslassstutzenhalter (74) im Lieferzustand.

6. Austragvorrichtung nach Anspruch 5,
**dadurch gekennzeichnet, dass**
korrespondierend zueinander Anschlagseinrichtungen (54a, 54b) am zweiten Gehäuseteilabschnitt (40) des ersten Gehäuseabschnitts (30) und am zweiten Gehäuseabschnitt (60) vorgesehen sind, die formschlüssig eine Trennung des zweiten Gehäuseteilabschnitts (40) zum zweiten Gehäuseabschnitt (60) verhindern.

7. Austragvorrichtung nach einem der vorstehenden Ansprüche,
**dadurch gekennzeichnet, dass**
am zweiten Gehäuseteilabschnitt (40) mindestens ein Sperrabschnitt (42) vorgesehen ist, der in eine Ausnehmung (72a) in einer Wandung des zweiten Gehäuseabschnitts (60) hineinragt oder durch diese Ausnehmung (72a) hindurchragt und eine Verlagerung des zweiten Gehäuseteilabschnitts (40) verhindert, solange eine Kappe (80) auf dem zweiten Gehäuseabschnitt (60) aufgesetzt ist.

8. Austragvorrichtung nach einem der vorstehenden Ansprüche,
**dadurch gekennzeichnet, dass**
die Gehäuseteilabschnitte (32, 40) zueinander korrespondierende Rastmittel (34, 44) aufweisen, durch die sie in zwei unterschiedlichen Relativlagen miteinander verrastbar sind.

9. Verfahren zur Montage und Inbetriebnahme einer Austragvorrichtung nach einem der vorstehenden Ansprüche,
**dadurch gekennzeichnet, dass**
- in einem Montageschritt die Gehäuseabschnitte (30, 60) soweit ineinander eingeschoben werden, dass die Kraftbeaufschlagungsfläche (32a) ausreichend weit vom Auslassstutzenhalter (74) beabstandet ist, dass der Auslassstutzen bei Anliegen des Pumpspenders (10) an der Kraftbeaufschlagungsfläche (32a) noch nicht in einer Endrelativlage relativ zum Auslassstutzenhalter (74) angeordnet ist, und
- in einem Inbetriebnahmeschritt nach der Auslieferung an den Patienten durch diesen durch fortgesetztes Einschieben der Gehäuseabschnitte (30, 60) ineinander der Auslassstutzen (16) bis in seine Endrelativlage relativ zum Auslassstutzen halter (74) gebracht wird, wobei zu diesem Zweck ein erster Gehäuseabschnitt (30) der beiden Gehäuseabschnitte (30, 60) als zweiteiliger Gehäuseabschnitt (30) mit einem ersten Gehäuseteilabschnitt (32), der die Kraftbeaufschlagungsfläche (32a) bzw. den Auslassstutzenhalter aufweist, und mit einem zweiten Gehäuseteilabschnitt (40), der gemeinsam mit dem zweiten Gehäuseabschnitt (60) die Führungseinrichtung (50) zum Zwecke der geführten Beweglichkeit der Gehäuseteile (30, 60) gegeneinander aufweist, ausgebildet ist und bei der Inbetriebnahme die beiden Gehäuseteilabschnitte (32, 40) gegeneinander verlagert werden.

## Claims

1. A discharging device for a liquid, more particularly a pharmaceutical liquid, adapted to accommodate and actuate a pump-type dispenser (10) and comprising
- a pump-type dispenser (10) having a liquid receptacle (12), a pumping mechanism (14), and an outlet port (16) comprising an outlet passage (16a), which pumping mechanism (14) can be actuated by depressing the outlet port (16) in the direction of the liquid receptacle (12),
- an accommodating region (66) for the at least partial accommodation of the pump-type dispenser (10), comprising
- an outlet port receiver (74) comprising a liquid passageway (76), said outlet port receiver (74) and said outlet port (16) being in a relative end position for creating a liquid-guiding and to the exterior leak-proof connection between said liquid passageway (76) and said outlet passage (16a), and
- a force-transferring surface (32a), disposed such that the displacement of said force-transferring surface (32a) in the direction (4a) of said outlet port receiver (74) also causes the liquid receptacle (12) of said pump-type dispenser (10) to be displaced in the direction of said outlet port receiver (74), and
- two mutually displaceable housing portions (30, 60) guided by a guiding mechanism, said outlet port receiver (74) being fixedly provided on one of said housing portions (60) and said force-transferring surface (32a) being fixedly provided on another of the housing portions (30),
wherein
- in a delivered state, said two housing portions (30, 60) and thus said outlet port receiver (74) and said force-transferring surface (32a) are spaced apart such that said outlet port (16) is disposed in a position relative to said outlet port receiver (74) which relative position is at a distance from said relative end position, and
- said housing portions (30, 60) are movable relatively to each other such that said outlet port (16) can be moved to its end position relative to said outlet port receiver (74) by displacement of said housing portions (30, 60),
**characterized in that**
a first housing portion (30) of said two housing portions (30, 60) is in the form of a two-membered housing portion (30),
wherein
- a first housing sub-portion (32) of said first housing portion (30) comprises said force-transferring surface (32a) or said outlet port receiver and a second housing sub portion (40) of said first housing portion (30) comprises, together with said second housing portion (60), said guiding mechanism (50) for the purpose of effecting guided movement of said housing portions (30, 60) relatively to each other, and
- said two housing sub-portions (32, 40) are mounted to another for being movable relatively to each other.

2. The discharging device as defined in claim 1,
**characterized in that**
said outlet port (16) and said outlet port receiver (74) have matching cylindrical portions, wherein the outside diameter (16b) of the cylindrical portion of said outlet port (16) and the inside diameter (74a) of the cylindrical portion of said outlet port receiver (74) are configured for a non-positive press fit connection in the relative end position, and wherein said cylindrical portions do not inter-engage in the delivered state.

3. The discharging device as defined in any of the preceding claims, **characterized in that**
a stop member is provided on said outlet port receiver (74) to define said relative end position.

4. The discharging device as defined in any of the preceding claims, **characterized in that**
- in the delivered state in said pump-type dispenser (10) there exists an atmosphere of inert gas, preferably a nitrogen inert gas atmosphere and/or
- said pumping mechanism (14) of said pump-type dispenser (10) communicates with said liquid receptacle (12) of said pump-type dispenser (10) via a connecting passageway, wherein in the delivered state the liquid is present only in said liquid receptacle (12) itself and in said connecting passageway.

5. The discharging device as defined in any of the preceding claims, **characterized in that**
said housing portions (30, 60) are formed such that the maximum distance between said force-transferring surface (32a) and said outlet port receiver (74) after an initial actuation is shorter than the distance between said force-transferring surface (32a) and said outlet port receiver (74) in the delivered state.

6. The discharging device as defined in claim 5,
**characterized in that**
matching stop members (54a, 54b) are provided on said second housing sub-portion (40) of said first housing portion (30) and on said second housing portion (60), which stop members (54a, 54b) positively prevent detachment of said second housing sub-portion (40) from said second housing portion (60).

7. The discharging device as defined in any of the preceding claims, **characterized in that**
on said second housing sub-portion (40) there is provided at least one locking portion (42) which protrudes into a recess (72a) in a wall of said second housing portion (60) or passes through this recess (72a), to prevent displacement of said second housing sub-portion (40) as long as a cap (80) is sitting on said second housing portion (60).

8. The discharging device as defined in any of the preceding claims, **characterized in that**
said housing sub-portions (32, 40) have matching locking means (34, 44), by means of which they can be locked together in two different relative positions.

9. A method for assembling and operating a discharging device as defined in any of the preceding claims,
**characterized in that**
- in an assembly step the housing portions (30, 60) are pushed into each other such that said force-transferring surface (32a) is spaced a sufficient length from said outlet port receiver (74) that when said pump-type dispenser (10) is in contact with said force-transferring surface (32a), said outlet port is still not disposed in a relative end position in relation to said outlet port receiver (74), and
- in an operating step following delivery to the patient, the patient causes said outlet port (16) to be moved to its relative end position in relation to said outlet port receiver (74) by pushing said housing portions (30, 60) further into each other, wherein for that purpose a first housing portion (30) of the two housing portions (30, 60) is configured as two-membered housing portion (30) having a first housing sub-portion (32) which comprises the force-transferring surface (32a) or the outlet port receiver, and having a second housing sub-portion (40) which together with the second housing portion (60) comprises the guiding mechanism (50) for the purpose of the guided movability of the housing parts (30, 60) relative to another, and during operating the two housing sub-portions (32, 40) are displaced relative to another.

## Revendications

1. Dispositif de sortie pour un liquide, en particulier un liquide pharmaceutique, destiné à contenir et à actionner un distributeur à pompe (10), avec
- un distributeur à pompe (10), qui comprend un réservoir de liquide (12), un système de pompe (14) et un embout de sortie (16) avec un canal de sortie (16a) et dont le système de pompe (14) peut être actionné par un enfoncement de l'embout de sortie (16) en direction du réservoir de liquide (12),
- une zone de logement (66) pour le logement au moins partiel du distributeur à pompe (10), avec
- un support d'embout de sortie (74) avec un canal de liquide (76), dans lequel le support d'embout de sortie (74) et l'embout de sortie (16) sont réalisés, dans une position relative d'extrémité, pour la liaison conduisant le liquide et étanche au liquide vers l'extérieur du canal de liquide (76) avec le canal de sortie (16a), et
- une face d'application de force (32a), qui est disposée de telle manière que, par un déplacement de la face d'application de force (32a) en direction (4a) du support d'embout de sortie (74), le réservoir de liquide (12) du distributeur à pompe (10) soit également déplacé en direction du support d'embout de sortie (74), et
- deux parties de boîtier (30, 60) déplaçables l'une par rapport à l'autre de façon guidée par un système de guidage, dans lequel le support d'embout de sortie (74) est prévu de façon fixe sur une des parties de boîtier (60) et dans lequel la face d'application de force (32a) est prévue de façon fixe sur une autre des parties de boîtier (30),
dans lequel
- dans un état de livraison, les deux parties de boîtier (30, 60) et dès lors le support d'embout de sortie (74) et la face d'application de force (32a), sont espacées l'une de l'autre, de telle manière que l'embout de sortie (16) soit disposé dans une position relative espacée de la position relative d'extrémité par rapport au support d'embout de sortie (74), et
- les parties de boîtier (30, 60) sont déplaçables l'une par rapport à l'autre, de telle manière que l'embout de sortie (16) puisse être amené dans sa position relative d'extrémité par rapport au support d'embout de sortie (74) par un déplacement des parties de boîtier (30, 60),
**caractérisé en ce que**
- une première partie de boîtier (30) des deux parties de boîtier (30, 60) est réalisée comme une partie de boîtier en deux pièces (30), dans lequel
- une première partie partielle de boîtier (32) de la première partie de boîtier (30) présente la face d'application de force (32a) ou le support d'embout de sortie et une deuxième partie partielle de boîtier (40) de la première partie de boîtier (30) présente, en commun avec la deuxième partie de boîtier (60), le système de guidage (50) destiné à assurer la mobilité guidée des parties de boîtier (30, 60) l'une par rapport à l'autre, et
- les deux parties partielles de boîtier (32, 40) sont agencées l'une à l'autre de façon déplaçable l'une par rapport à l'autre.

2. Dispositif de sortie selon la revendication 1, **caractérisé en ce que** l'embout de sortie (16) et le support d'embout de sortie (74) présentent des parties cylindriques se correspondant mutuellement, dans lequel le diamètre extérieur (16b) de la partie cylindrique de l'embout de sortie (16) et le diamètre intérieur (74a) de la partie cylindrique du support d'embout de sortie (74) sont réalisés pour assurer une liaison ajustée serrée dans la position relative d'extrémité et dans lequel les parties cylindriques ne sont pas engagées l'une avec l'autre dans l'état de livraison.

3. Dispositif de sortie selon l'une quelconque des revendications précédentes, **caractérisé en ce qu'**il est prévu sur le support d'embout de sortie (74) une butée définissant la position relative d'extrémité.

4. Dispositif selon l'une quelconque des revendications précédentes, **caractérisé en ce que**
- dans l'état de livraison, il existe dans le distributeur à pompe (10) une atmosphère de gaz protecteur, de préférence une atmosphère de gaz protecteur d'azote, et/ou
- le système de pompe (14) du distributeur à pompe (10) est relié par un canal de liaison au réservoir de liquide (12) du distributeur à pompe (10), dans lequel le liquide, dans l'état de livraison, est disposé uniquement dans le réservoir de liquide (12) lui-même et dans le canal de liaison.

5. Dispositif de sortie selon l'une quelconque des revendications précédentes, **caractérisé en ce que** les parties de boîtier (30, 60) sont réalisées de telle manière que l'espacement maximal entre la face d'application de force (32a) et le support d'embout de sortie (74) après un premier actionnement est plus petit que l'espacement entre la face d'application de force (32a) et le support d'embout de sortie (74) dans l'état de livraison.

6. Dispositif de sortie selon la revendication 5, **caractérisé en ce qu'**il est prévu des systèmes de butée se correspondant mutuellement (54a, 54b) sur la deuxième partie partielle de boîtier (40) de la première partie de boîtier (30) et sur la deuxième partie de boîtier (60), qui empêchent par emboîtement une séparation de la deuxième partie partielle de boîtier (40) de la deuxième partie de boîtier (60).

7. Dispositif de sortie selon l'une quelconque des revendications précédentes, **caractérisé en ce qu'**il est prévu sur la deuxième partie partielle de boîtier (40) au moins une partie de blocage (42), qui pénètre dans un évidement (72a) dans une paroi de la deuxième partie de boîtier (60) ou qui passe à travers cet évidement (72a) et empêche un déplacement de la deuxième partie partielle de boîtier (40), aussi longtemps qu'un capuchon (80) est posé sur la deuxième partie de boîtier (60).

8. Dispositif de sortie selon l'une quelconque des revendications précédentes, **caractérisé en ce que** les parties partielles de boîtier (32, 40) présentent des moyens d'encliquetage se correspondant mutuellement (34, 44), par lesquels elles peuvent être encliquetées l'une avec l'autre dans deux positions relatives différentes.

9. Procédé de montage et de mise en service d'un dispositif de sortie selon l'une quelconque des revendications précédentes, **caractérisé en ce que**:
- dans une étape de montage, on insère les parties de boîtier (30, 60) l'une dans l'autre à un point tel que la face d'application de force (32a) soit suffisamment espacée du support d'embout de sortie (74) pour que l'embout de sortie ne se trouve pas encore dans une position relative d'extrémité par rapport au support d'embout de sortie (74) lors de l'application du distributeur à pompe (10) sur la face d'application de force (32a), et
- dans une étape de mise en service après la livraison au patient, on amène l'embout de sortie (16) jusque dans sa position relative d'extrémité par rapport au support d'embout de sortie (74) en poursuivant l'insertion des parties de boîtier (30, 60) l'une dans l'autre par le patient, dans lequel une première partie de boîtier (30) des deux parties de boîtier (30, 60) est à cet effet réalisée sous la forme d'une partie de boîtier en deux pièces (30), avec une première partie partielle de boîtier (32), qui présente la face d'application de force (32a) ou le support d'embout de sortie, et avec une deuxième partie partielle de boîtier (40) qui présente, en commun avec la deuxième partie de boîtier (60), le système de guidage (50) destiné à assurer la mobilité guidée des parties de boîtier (30, 60) l'une par rapport à l'autre, et on déplace les deux parties partielles de boîtier (32, 40) l'une par rapport à l'autre lors de la mise en service.
